# EUROPEAN PATENT APPLICATION

(11) **EP 2 850 952 A1**
(43) Date of publication of application: **25.03.2015**
(21) Application number: 13790582.4
(22) Date of filing: 17.05.2013
(51) Int. Cl.: A23L 1/221, A23L 1/31

(54) **ENZYME PREPARATION FOR MODIFYING FOOD MATERIAL**

(30) Priority: 17.05.2012 JP 2012113746
(71) Applicant: Nagase ChemteX Corporation, Osaka-shi, Osaka 550-8668 (JP); Nagase & Co., Ltd., Osaka-shi Osaka 550-8668 (JP)
(72) Inventor: MAKINO, Yosuke, Kobe-shi Hyogo 651-2241 (JP); NISHIMOTO, Yukifumi, Kobe-shi Hyogo 651-2241 (JP)
(74) Representative: Koepe & Partner Patentanwälte
(86) International application number: PCT/JP2013/063777
(87) International publication number: WO 2013/172447

(57) **Abstract**

An enzyme preparation for suppressing bitterness of food of the present invention contains phospholipase and, if necessary, further contains protease. A method for suppressing bitterness of food of the invention includes treating a food material with a food bitterness suppressor containing the enzyme preparation. A method for producing a processed food product of the invention includes treating a food material with a food bitterness suppressor containing the enzyme preparation. Food treated with the enzyme preparation for suppressing bitterness of food of the invention tastes less bitter even when cooked after long-term preservation. Furthermore, an enzyme preparation for suppressing bitterness of food and for, if necessary, tenderizing food can be provided.

## Description

### Technical Field

The present invention relates to an enzyme preparation for modifying a food material. More specifically, the invention relates to an enzyme preparation for suppressing bitterness of food or an enzyme preparation for tenderizing food. The present invention further relates to a method for suppressing bitterness of food or a method for tenderizing food and a method for producing a processed food product.

### Background Art

There are various known methods for modifying a food material by treating the food material with an enzyme. For example, Patent Document 1 describes a method for tenderizing meat, which includes contacting meat with a tenderizing-effective amount of non-heat resistant protease. Patent Document 2 describes a method for producing cholesterol-reduced meat by treating meat with phospholipase B, phospholipase C, or phospholipase D.

### Prior Art Documents

### Patent Document

Patent Document 1 Japanese Laid-Open Patent Publication No. 2003-508084
Patent Document 2 Japanese Laid-Open Patent Publication No. H05-049414

### Summary of Invention

### Problems to be Solved by the Invention

It is an object of the present invention to provide an enzyme preparation for suppressing bitterness of food. Further, it is another object of the present invention to provide an enzyme preparation for suppressing bitterness of food and for, if necessary, tenderizing food.

### Means for Solving the Problem

The inventors found that bitterness of food can be suppressed by treating a food material with phospholipase. Furthermore, they found that bitterness of food can be suppressed and the food can be tenderized by treating a food material with phospholipase and protease.

The present invention provides an enzyme preparation for suppressing bitterness of food, containing phospholipase.

In one embodiment, the enzyme preparation further contains protease.

In one embodiment, the food is edible meat.

In one embodiment, the bitterness is derived from peptide generated due to an action of protease on the food material.

In one embodiment, the phospholipase is phospholipase D.

In one embodiment, the phospholipase is contained in a proportion of 60 to 1500 units (U) with respect to 100 units (U) of the protease.

The present invention further provides a method for suppressing bitterness of food, which includes treating a material of the food with a food bitterness suppressor containing the above-described enzyme preparation.

The present invention further provides a method for producing a processed food product, which includes a step of treating a food material with a food bitterness suppressor containing the enzyme preparation.

The present invention further provides a processed food product, containing a food material treated with a food bitterness suppressor containing the enzyme preparation.

### Effects of Invention

The present invention can provide an enzyme preparation for suppressing bitterness of food. Food treated with the enzyme preparation for suppressing bitterness of food of the present invention does not taste bitter even when cooked after long-term preservation. The present invention can further provide an enzyme preparation for suppressing bitterness of food and for, if necessary, tenderizing food.

### Mode for Carrying out the Invention

An enzyme preparation for suppressing bitterness of food of the present invention contains phospholipase.

There is no particular limitation on the food, and examples thereof include meat products, rice products, noodles, confectioneries, vegetable-derived or fruit-derived beverages, processed aquatic products, processed livestock products (egg products, dairy products, etc.), fermented foods, and extract seasonings (meat extract, vegetable extract, yeast extract, etc.).

The enzyme preparation of the present invention is used for treating a food material. The food material in the present invention refers to a material of the food as mentioned above, having properties on which the enzyme can act. The food material is, for example, a "raw" material that is not subjected to heat treatment or the like. The material is preferably rich in peptide or protein. There is no particular limitation on the food material, and examples thereof include edible meats, cereals (flour, etc.), vegetables and fruits, aquatic products, livestock products (eggs, milk, etc.), supplements, or nutrient components (peptide and amino acid), and mixtures thereof.

There is no particular limitation on the edible meat, and examples thereof include beef, pork, chicken, horse meat, sheep meat, deer meat, goat meat, rabbit meat, duck meat, goose meat, turkey meat, and quail meat. There is no particular limitation on parts of the meat, and examples thereof include leg, breast, shoulder, chuck shoulder, loin, rib, tenderloin, sirloin, round, shank, and various internal organs. There is no particular limitation on the form of the meat, and examples thereof include dressed carcass, cut meat, and dressed meat (sliced, diced, chopped (trimmings), ground meat, etc.). The edible meat may be preserved by refrigeration or preserved by freezing, or may be thawed after being preserved by freezing.

The suppressing bitterness of food in the present invention is to suppress bitterness of food after cooking, by causing an enzyme preparation to act on a food material.

There is no particular limitation on the phospholipase, and examples thereof include phospholipase A, phospholipase B, phospholipase C, and phospholipase D. Phospholipase C and phospholipase D are preferred, and phospholipase D is more preferred. The phospholipases may be used alone or in a combination of two or more. Furthermore, the phospholipase may be commercially available phospholipase, or may be phospholipase prepared from a phospholipase-producing microorganism. There is no particular limitation on the commercially available phospholipase A, and examples thereof include Phospholipase A (manufactured by Mitsubishi-Kagaku Foods Corporation), Maxapal A2 (manufactured by DSM), Lipomod 699L (manufactured by Kyowa Hakko Bio Co., Ltd.), PLA2 Nagase (manufactured by Nagase ChemteX Corporation), and Lecitase (manufactured by Novozymes). There is no particular limitation on the commercially available phospholipase B, and examples thereof include Phospholipase B (P8914) (manufactured by Sigma-Aldrich). There is no particular limitation on the commercially available phospholipase C, and examples thereof include Purifine (manufactured by Verenium), Phospholipase C (P6621) (manufactured by Sigma-Aldrich), Phospholipase C (P7633) (manufactured by Sigma-Aldrich), and Phospholipase C (P4039) (manufactured by Sigma-Aldrich). There is no particular limitation on the commercially available phospholipase D, and examples thereof include Phospholipase D (manufactured by Meito Sangyo Co., Ltd. and Asahi Kasei Corporation), Phospholipase D (P8023) (manufactured by Sigma-Aldrich), Phospholipase D (P4912) (manufactured by Sigma-Aldrich), and Phospholipase D (P7758) (manufactured by Sigma-Aldrich). There is no particular limitation on the phospholipase D-producing microorganism, and examples thereof include microorganisms belonging to actinomycetes of *Actinomadura, Streptomyces, Streptoverticillium, Micromonospora, Nocardia, Nocardiopsis, Actinomadura,* and the like, more specifically including *Streptomyces antibioticus, Streptomyces acidomyceticus, Streptomyces chromofuscus, Streptomyces* sp. AA586, *Streptomyces* sp. PMF, *Streptoverticillium cinnamoneum, Streptomyces cinnamoneum* IFO 12852, *Micromonospora chalcea* ATCC12452, *Nocardia mediterranei* IFO 13142, *Nocardiopsis dassonvillei* IFO 13908, and *Actinomadura libanotica* IFO 14095. There is no particular limitation on the phospholipase D-producing animal or plant, and examples thereof include cabbage. There is no particular limitation on the method for preparing phospholipase D from the phospholipase D-producing microorganism or the phospholipase D-producing animal or plant, and methods commonly used by those skilled in the art are used.

The enzyme preparation for suppressing bitterness of food of the present invention may further contain protease. If the food material is treated with the enzyme preparation of the present invention, bitterness generated in the food material due to the action of the protease can be suppressed by the phospholipase. It seems that the bitterness is derived from peptide generated due to the action of the protease on the food material.

There is no particular limitation on the protease, and examples thereof include *Bacillus subtillis-*derived protease (various bioprases, manufactured by Nagase ChemteX Corporation, etc.), papain, bromelain, actinidin, ficin, protease derived from *Aspergillus* (Denatyme, manufactured by Nagase ChemteX Corporation, etc.), *Aspergillus oryzae*-derived protease, and *Aspergillus niger-*derived protease. The protease may be commercially available protease, or may be protease prepared from a protease-producing microorganism or a protease-producing animal or plant. There is no particular limitation on the commercially available protease, and examples thereof include various bioprases such as Bioprase OP (manufactured by Nagase ChemteX Corporation), purified papain (manufactured by Nagase ChemteX Corporation), and Denatyme (manufactured by Nagase ChemteX Corporation). There is no particular limitation on the protease-producing microorganism, and examples thereof include *Bacillus subtillis* and other microorganisms of *Bacillus; Aspergillus oryzae, Aspergillus niger,* and other microorganisms of *Aspergillus*; and microorganisms of *Streptomyces.* There is no particular limitation on the protease-producing animal or plant, and examples thereof include papaya, pineapple, kiwi fruit, and fig. There is no particular limitation on the method for preparing protease from the protease-producing microorganism or the protease-producing animal or plant, and methods commonly used by those skilled in the art are used.

There is no particular limitation on the proportion of phospholipase contained with respect to protease in the enzyme preparation of the present invention, but it is preferably 60 to 1500 units (U), more preferably 80 to 1500 units (U), and even more preferably 350 to 1500 units (U), with respect to 100 units (U) of protease.

Note that the activity of protease (units: U) is determined as follows. When 1 mL of enzyme solution is added to 5 mL of 0.6% milk casein (pH 7.5, M/25 phosphate buffer solution) and reacted at 30°C for 10 minutes, the amount of enzyme releasing, as a TCA soluble component, Folin coloring development corresponding to 1 µg of tyrosine in 1 minute is taken as 1 U.

The activities of phospholipases A and B (units: U) are determined as follows. Free fatty acid produced when hydrolyzing soybean lecithin as a substrate is quantitated using a commercially available free fatty acid quantitation reagent kit Determiner NEFA755 (manufactured by Kyowa Medex Co., Ltd.). The amount of enzyme releasing 1 µmol of fatty acid in 1 minute is taken as 1 U.

The activity of phospholipase C (units: U) is determined as follows. Commercially available alkaline phosphatase (manufactured by Takara Bio Inc.) is added to phosphorylcholine produced when hydrolyzing soybean lecithin as a substrate, and phosphoric acid released is quantitated using a commercially available BIOMOL GREEN (manufactured by BIOMOL Research Laboratories). The amount of enzyme releasing 1 µmol of phosphorylcholine in 1 minute is taken as 1 U.

The activity of phospholipase D (units: U) is determined as follows. Choline produced when hydrolyzing phosphatidylcholine as a substrate is quantitated using a commercially available Cholinesterase Kit-NC (289-75181, manufactured by Wako Pure Chemical Industries, Ltd.). Here, the resultant choline produces red quinone pigment by choline oxidase, peroxidase, or the like in the kit. The amount of enzyme releasing 1 µmol of choline in 1 minute is taken as 1 U.

The phospholipase and the protease may be mixed in advance, or may not be mixed. That is, the phospholipase and the protease may be separately provided as kit preparations.

The enzyme preparation of the present invention can be used for suppressing bitterness of food. Furthermore, another enzyme preparation of the present invention can be used for suppressing bitterness of food and for tenderizing food. If the food material is treated with the enzyme preparation of the present invention, food or a processed food product whose bitterness has been suppressed and, if necessary, that has been tenderized can be produced.

The enzyme preparation of the present invention may contain any amount of enzyme other than the phospholipase and the protease, as long as the effects of the present invention are not inhibited. There is no particular limitation on the enzyme, and examples thereof include amylase, glucanase, and lipase.

The enzyme preparation of the present invention may contain any amount of component commonly contained in an enzyme preparation, such as an excipient, as long as the effects of the present invention are not inhibited. There is no particular limitation on the excipient, and examples thereof include sugars such as glucose, lactose, and trehalose; sugar alcohols such as maltitol and sorbitol; polysaccharides such as dextrin, starch, and pectin; gums, and inorganic salts (sodium chloride, etc.).

There is no particular limitation on the form of the enzyme preparation of the present invention, and examples thereof include powder and liquid. In the case of liquid, there is no particular limitation on the solvent or the dispersion medium, as long as the enzyme functions and there is no hygienic problem, but it is preferably water. If the solvent or the dispersion medium is water, there is no particular limitation on the pH, but it is preferably 5 to 10, more preferably 6 to 9. The amount of phospholipase and the amount of protease in the enzyme preparation of the present invention are set as appropriate.

The method for suppressing bitterness of food in accordance with the present invention, includes a step of treating the food material with a food bitterness suppressor containing the enzyme preparation as described above.

The food bitterness suppressor may contain, in addition to the enzyme preparation, salts, seasonings, spices, food additives (polysaccharides, trisodium citrate, sodium bicarbonate, etc.).

There is no particular limitation on the form of the food bitterness suppressor, and examples thereof include powder and liquid. In the case of liquid, there is no particular limitation on the solvent or the dispersion medium, as long as the enzyme functions and there is no hygienic problem, but it is preferably water. If the solvent or the dispersion medium is water, there is no particular limitation on the pH, but it is preferably 5 to 10, more preferably 6 to 9.

There is no particular limitation on the form for treating the food material with the food bitterness suppressor, and examples thereof include a form in which the food bitterness suppressor is mixed with, applied to, sprayed onto, or injected into the food material (such as by sticking the tip of a single-needle or multi-needle injector into the food material, and supplying an appropriate amount of food bitterness suppressor inside the injector syringe into the food material) into the food material and a form in which the food material is immersed in a liquid containing the food bitterness suppressor.

There is no particular limitation on the amount of protease used for treating the food material, but it is preferably 10 to 2000 units (U), more preferably 30 to 1000 units (U), with respect to 100 g of the food material.

There is no particular limitation on the amount of phospholipase used for treating the food material, but it is preferably 30 to 5000 units (U), more preferably 100 to 4000 units (U), with respect to 100 g of the food material.

There is no particular limitation on the temperature for treating the food material, and it is, for example, 0 to 25°C, preferably 0 to 15°C.

There is no particular limitation on the time for treating the food material, and it is set as appropriate according to the type of food material, the form of food material, and the like. For example, it is 1 hour to 30 days, preferably 2 hours to 16 days, and more preferably 2 days to 16 days.

The treatment with the phospholipase and the treatment with the protease may be simultaneously performed or may be sequentially performed. If the treatments are sequentially performed, there is no particular limitation on the treatment orders.

The method for producing a processed food product in accordance with the present invention, includes a step of treating the food material with a food bitterness suppressor containing the enzyme preparation as described above.

The processed food product in the present invention is a product obtained by processing food, and is a product finally subjected to processing such as roasting, simmering, boiling, steaming, smoking, or the like. There is no particular limitation on the processed food product, and examples thereof include hamburg steak, deep-fried chicken, ham, sausage, meatball, yeast extract, meat extract, vegetable extract, cookie, gum, vegetable juice, rice ball, instant noodles in cup, fermented soybeans, yoghurt, fish paste, tubular roll of fish paste, and supplements.

The food treated with the food bitterness suppressor is cooked as appropriate. There is no particular limitation on the cooking, and examples thereof include cooking with heat, such as roasting, simmering, boiling, steaming, smoking, and the like. Before the cooking, the food may be preserved by freezing and then be thawed. The food or the processed food product after the cooking tastes less bitter, preferably does not taste bitter.

### Examples

Hereinafter, the present invention will be described further in detail by way of examples, but the present invention is not limited thereto.

### (Preparation Example)

An enzyme preparation (powder) containing protease (Bioprase: Bioprase OP manufactured by Nagase ChemteX Corporation, or Papain: purified papain for food manufactured by Nagase ChemteX Corporation) and phospholipase D (PLD: *Streptomyces cinnamoneum*-derived PLD, manufactured by Nagase ChemteX Corporation) in the formulations in Tables 1 to 3 below was prepared following a regular method using an excipient (Ako salt R, manufactured by Ako Kaisui Co., Ltd.), and 1.25 g of each enzyme preparation was dissolved in 100 g of water to give a test enzyme solution. In Table 3, trehalose or cyclodextrin known as a bitterness masking substance was added. A1 to A3 are 100 g of water (control).

**Table 1**

| Enzyme Preparation | Bioprase | PLD |
|---|---|---|
| A1 | - | - |
| B1 | 5600U/g | - |
| C1 | 2800U/g | - |
| D1 | 1400U/g | - |
| E1 | 700U/g | - |
| F1 | 5600U/g | 5000U/g |
| G1 | 2800U/g | 5000U/g |
| H1 | 1400U/g | 5000U/g |
| I1 | 700U/g | 5000U/g |

**Table 2**

| Enzyme Preparation | Papain | PLD |
|---|---|---|
| A2 | - | - |
| B2 | 5600U/g | - |
| C2 | 2800U/g | - |
| D2 | 1400U/g | - |
| E2 | 700U/g | - |
| F2 | 5600U/g | 5000U/g |
| G2 | 2800U/g | 5000U/g |
| H2 | 1400U/g | 5000U/g |
| I2 | 700U/g | 5000U/g |

**Table 3**

| Enzyme Preparation | Bioprase | Papain | PLD | Trehalose | Cyclodextrin |
|---|---|---|---|---|---|
| A3 | - | - | - | - | - |
| B3 | 5600U/g | - | - | - | - |
| C3 | 5600U/g | - | 5000U/g | - | - |
| D3 | 5600U/g | - | - | 80% by mass | - |
| E3 | 5600U/g | - | - | - | 80% by mass |
| F3 | - | 5600U/g | - | - | - |
| G3 | - | 5600U/g | 5000U/g | - | - |
| H3 | - | 5600U/g | - | 80% by mass | - |
| I3 | - | 5600U/g | - | - | 80% by mass |

### (Test Example)

### (Test Example 1: Test of Hamburg Steak containing Ground Pork Meat)

### Hamburg steak formulation

| | |
|---|---|
| Ground pork meat | 500 g |
| Salt | 5 g |
| Pepper | 2 g |
| Test enzyme solution | 20 mL |

These materials were mixed to prepare a raw hamburg steak. The raw hamburg steak was preserved in a refrigerator (5°C) for 2 days, and, after heating, the hardness and the bitterness of the meat were evaluated. On the other hand, the raw hamburg steak was preserved in a freezer (-15°C) for 16 days and thawed (at ambient temperature overnight), and, after heating, the hardness and the bitterness of the meat were evaluated. The heating method was such that, in order to uniformly apply heat, the raw hamburg steak was wrapped in plastic wrap and heated using a microwave oven (600 W) for 5 minutes. The results are shown in Tables 4 to 6. The evaluation method was as follows.

### (Evaluation Method)

Nine people performed evaluation by tasting.

Regarding the hardness, each person evaluated the food on a 5-grade scale below, and an average of the evaluations by the nine people was obtained.

Regarding the bitterness, each person evaluated whether the food did not taste bitter (O) or tasted bitter (x), where the case in which a majority did not detect a bitter taste was indicated as "O" and the case in which a majority detected a bitter taste was indicated as "x".

### (Evaluation Criteria for Hardness)

- -:: Hardness of control
- +:: Slightly tenderer than control
- ++:: Moderately tender
- +++:: Considerably tender
- ++++:: Very tender

**Table 4**

| Enzyme Preparation | After preserved in a refrigerator for 2 days | | After preserved in a refrigerator for 16 days | |
|---|---|---|---|---|
| | Hardness | Bitterness | Hardness | Bitterness |
| A1 | - | ○ | - | ○ |
| B1 | ++ | × | ++ | × |
| C1 | ++ | × | ++ | × |
| D1 | ++ | × | ++ | × |
| E1 | + | × | + | × |
| F1 | ++ | ○ | ++ | ○ |
| G1 | ++ | ○ | ++ | ○ |
| H1 | ++ | ○ | ++ | ○ |
| I1 | + | ○ | + | ○ |

**Table 5**

| Enzyme Preparation | After preserved in a refrigerator for 2 days | | After preserved in a refrigerator for 16 days | |
|---|---|---|---|---|
| | Hardness | Bitterness | Hardness | Bitterness |
| A2 | - | ○ | - | ○ |
| B2 | +++ | × | ++++ | × |
| C2 | +++ | × | +++ | × |
| D2 | ++ | × | +++ | × |
| E2 | + | × | ++ | × |
| F2 | +++ | ○ | ++++ | ○ |
| G2 | +++ | ○ | +++ | ○ |
| H2 | ++ | ○ | +++ | ○ |
| I2 | + | ○ | ++ | ○ |

**Table 6**

| Enzyme Preparation | After preserved in a refrigerator for 2 days | | After preserved in a refrigerator for 16 days | |
|---|---|---|---|---|
| | Hardness | Bitterness | Hardness | Bitterness |
| A3 | - | ○ | - | ○ |
| B3 | ++ | × | ++ | × |
| C3 | ++ | ○ | ++ | ○ |
| D3 | ++ | × | ++ | × |
| E3 | ++ | × | ++ | × |
| F3 | +++ | × | ++++ | × |
| G3 | +++ | ○ | ++++ | ○ |
| H3 | +++ | × | ++++ | × |
| I3 | +++ | × | ++++ | × |

As seen from Tables 4 and 5, the hamburg steaks to which only protease was added had tender meat texture, but tasted bitter. However, when phospholipase D was further added thereto, hamburg steaks having tender meat texture and not tasting bitter could be provided.

As seen from Table 6, even when trehalose or cyclodextrin known as a bitterness masking substance was further added thereto, no hamburg steak having tender meat texture and not tasting bitter could be provided.

### (Test Example 2: Test of Pork Leg Meat Immersion)

Eighty mL of test enzyme solution was mixed with 920 mL of 1% (w/v) sodium bicarbonate solution to prepare a test solution. Then, 400 g of pork leg meat was immersed in the test solution, preserved in a refrigerator (5°C) for 2 days, and, after heating, the hardness and the bitterness of the meat were evaluated. On the other hand, the pork leg meat was immersed in the test solution, preserved in a refrigerator (5°C) for 2 days, and, then, after removal of the test solution, further preserved in a freezer (-15°C) for 16 days and thawed (at ambient temperature overnight), and, after heating, the hardness and the bitterness of the meat were evaluated. The heating method was such that, in order to uniformly apply heat, the pork leg meat was wrapped in plastic wrap and heated using a microwave oven (600 W) for 5 minutes. The results are shown in Tables 7 to 9. The evaluation was performed as in Test Example 1.

**Table 7**

| Enzyme Preparation | After preserved in a refrigerator for 2 days | | After preserved in a refrigerator for 16 days | |
|---|---|---|---|---|
| | Hardness | Bitterness | Hardness | Bitterness |
| A1 | - | ○ | - | ○ |
| B1 | ++ | × | +++ | × |
| C1 | ++ | × | ++ | × |
| D1 | ++ | × | ++ | × |
| E1 | ++ | × | ++ | × |
| F1 | ++ | ○ | +++ | ○ |
| G1 | ++ | ○ | ++ | ○ |
| H1 | ++ | ○ | ++ | ○ |
| I1 | ++ | ○ | ++ | ○ |

**Table 8**

| Enzyme Preparation | After preserved in a refrigerator for 2 days | | After preserved in a refrigerator for 16 days | |
|---|---|---|---|---|
| | Hardness | Bitterness | Hardness | Bitterness |
| A2 | - | ○ | - | ○ |
| B2 | ++++ | × | ++++ | × |
| C2 | +++ | × | +++ | × |
| D2 | ++ | × | ++ | × |
| E2 | ++ | × | ++ | × |
| F2 | ++++ | ○ | ++++ | ○ |
| G2 | +++ | ○ | +++ | ○ |
| H2 | ++ | ○ | ++ | ○ |
| I2 | ++ | ○ | ++ | ○ |

**Table 9**

| Enzyme Preparation | After preserved in a refrigerator for 2 days | | After preserved in a refrigerator for 16 days | |
|---|---|---|---|---|
| | Hardness | Bitterness | Hardness | Bitterness |
| A3 | - | ○ | - | ○ |
| B3 | ++ | × | ++ | × |
| C3 | ++ | ○ | ++ | ○ |
| D3 | ++ | × | ++ | × |
| E3 | ++ | × | ++ | × |
| F3 | ++++ | × | ++++ | × |
| G3 | ++++ | ○ | ++++ | ○ |
| H3 | ++++ | × | ++++ | × |
| I3 | ++++ | × | ++++ | × |

As seen from Tables 7 and 8, the pork leg meats to which only protease was added had tender meat texture, but tasted bitter. However, when phospholipase D was further added thereto, pork leg meats having tender meat texture and not tasting bitter could be provided.

As seen from Table 9, even when trehalose or cyclodextrin known as a bitterness masking substance was further added thereto, no pork leg meat having tender meat texture and not tasting bitter could be provided.

### (Test Example 3: Test of Beef Leg Meat Injection)

Eighty mL of test enzyme solution was mixed with 920 mL of 1% (w/v) sodium bicarbonate and 0.2% (w/v) xanthan gum solution to prepare a test solution. Then, the test solution was injected using an injector into 400 g of beef leg meat, the beef leg meat was preserved in a refrigerator (5°C) for 2 days, and, after heating, the hardness and the bitterness of the meat were evaluated. On the other hand, the test solution was injected using an injector into the beef leg meat, the beef leg meat was preserved in a refrigerator (5°C) for 2 days, and further preserved in a freezer (-15°C) for 16 days and thawed (at ambient temperature overnight), and, after heating, the hardness and the bitterness of the meat were evaluated. The heating method was such that, in order to uniformly apply heat, the beef leg meat was wrapped in plastic wrap and heated using a microwave oven (600 W) for 5 minutes. The results are shown in Tables 10 to 12. The evaluation was performed as in Test Example 1.

**Table 10**

| Enzyme Preparation | After preserved in a refrigerator for 2 days | | After preserved in a refrigerator for 16 days | |
|---|---|---|---|---|
| | Hardness | Bitterness | Hardness | Bitterness |
| A1 | - | ○ | - | ○ |
| B1 | +++ | × | ++++ | × |
| C1 | +++ | × | ++++ | × |
| D1 | ++ | × | ++++ | × |
| E1 | ++ | × | ++ | × |
| F1 | +++ | ○ | ++++ | ○ |
| G1 | +++ | ○ | ++++ | ○ |
| H1 | ++ | ○ | ++++ | ○ |
| I1 | ++ | ○ | ++ | ○ |

**Table 11**

| Enzyme Preparation | After preserved in a refrigerator for 2 days | | After preserved in a refrigerator for 16 days | |
|---|---|---|---|---|
| | Hardness | Bitterness | Hardness | Bitterness |
| A2 | - | ○ | - | ○ |
| B2 | +++ | × | +++ | × |
| C2 | +++ | × | +++ | × |
| D2 | ++ | × | +++ | × |
| E2 | ++ | × | ++ | × |
| F2 | +++ | ○ | +++ | ○ |
| G2 | +++ | ○ | +++ | ○ |
| H2 | ++ | ○ | +++ | ○ |
| I2 | ++ | ○ | ++ | ○ |

**Table 12**

| Enzyme Preparation | After preserved in a refrigerator for 2 days | | After preserved in a refrigerator for 16 days | |
|---|---|---|---|---|
| | Hardness | Bitterness | Hardness | Bitterness |
| A3 | - | ○ | - | ○ |
| B3 | +++ | × | ++++ | × |
| C3 | +++ | ○ | ++++ | ○ |
| D3 | +++ | × | ++++ | × |
| E3 | +++ | × | ++++ | × |
| F3 | +++ | × | +++ | × |
| G3 | +++ | ○ | +++ | ○ |
| H3 | +++ | × | +++ | × |
| I3 | +++ | × | +++ | × |

As seen from Tables 10 and 11, the beef leg meats to which only protease was added had tender meat texture, but tasted bitter. However, when phospholipase D was further added thereto, beef leg meats having tender meat texture and not tasting bitter could be provided.

As seen from Table 12, even when trehalose or cyclodextrin known as a bitterness masking substance was further added thereto, no beef leg meat having tender meat texture and not tasting bitter could be provided.

### (Test Example 4: Test of Chicken Breast Meat Immersion)

The test was performed as in Test Example 2, except that chicken breast meat was used instead of pork leg meat. The results are shown in Tables 13 to 15.

**Table 13**

| Enzyme Preparation | After preserved in a refrigerator for 2 days | | After preserved in a refrigerator for 16 days | |
|---|---|---|---|---|
| | Hardness | Bitterness | Hardness | Bitterness |
| A1 | - | ○ | - | ○ |
| B1 | ++ | × | ++ | × |
| C1 | + | × | ++ | × |
| D1 | + | × | ++ | × |
| E1 | + | × | + | × |
| F1 | ++ | ○ | ++ | ○ |
| G1 | + | ○ | ++ | ○ |
| H1 | + | ○ | ++ | ○ |
| I1 | + | ○ | + | ○ |

**Table 14**

| Enzyme Preparation | After preserved in a refrigerator for 2 days | | After preserved in a refrigerator for 16 days | |
|---|---|---|---|---|
| | Hardness | Bitterness | Hardness | Bitterness |
| A2 | - | ○ | - | ○ |
| B2 | ++ | × | ++ | × |
| C2 | ++ | × | ++ | × |
| D2 | ++ | × | ++ | × |
| E2 | + | × | + | × |
| F2 | ++ | ○ | ++ | ○ |
| G2 | ++ | ○ | ++ | ○ |
| H2 | ++ | ○ | ++ | ○ |
| I2 | + | ○ | + | ○ |

**Table 15**

| Enzyme Preparation | After preserved in a refrigerator for 2 days | | After preserved in a refrigerator for 16 days | |
|---|---|---|---|---|
| | Hardness | Bitterness | Hardness | Bitterness |
| A3 | - | ○ | - | ○ |
| B3 | ++ | × | ++ | × |
| C3 | ++ | ○ | ++ | ○ |
| D3 | ++ | × | ++ | × |
| E3 | ++ | × | ++ | × |
| F3 | ++ | × | ++ | × |
| G3 | ++ | ○ | ++ | ○ |
| H3 | ++ | × | ++ | × |
| I3 | ++ | × | ++ | × |

As seen from Tables 13 and 14, the chicken breast meats to which only protease was added had tender meat texture, but tasted bitter. However, when phospholipase D was further added thereto, chicken breast meats having tender meat texture and not tasting bitter could be provided.

As seen from Table 15, even when trehalose or cyclodextrin known as a bitterness masking substance was further added thereto, no chicken breast meat having tender meat texture and not tasting bitter could be provided.

### (Test Example 5: Bitterness Masking Effect on Various Edible Peptides)

In this test, 4.4 mg (2500 U) of phospholipase D powder (PLD: *Streptomyces cinnamoneum*-derived PLD, manufactured by Nagase ChemteX Corporation) was dissolved in 1200 mL of tap water to give a test enzyme solution (2) having 3.7 µg/mL of a PLD concentration.

Then, 2 g of each peptide product (powder) shown in Table 16 was weighed and dissolved in 40 mL of either the test enzyme solution (2) (containing PLD) or tap water (not containing PLD), and the solution was freeze-dried. Note that the group using the enzyme solution (2) was freeze-dried after the peptide product was dissolved and reacted with the enzyme (25°C, 3 hours).

Ten evaluators performed taste test on the obtained freeze-dried samples containing PLD and not containing PLD for each peptide product, and judged which of the freeze-dried samples containing PLD and the freeze-dried samples not containing PLD tasted bitter. If an evaluator detected a bitter taste in both samples without much difference, the evaluator judged that both samples tasted bitter. The results are shown in Table 16.

**Table 16**

| Sample No. | Peptide Product | Number of Evaluators who Detected a Bitter Taste | | |
|---|---|---|---|---|
| | | Not containing of PLD | Containing of PLD | Both |
| M1 | Chicken collagen peptide (C-LAP; NH Foods Ltd.) | 6 | 2 | 2 |
| M2 | Silkpeptide (Cosmo Shokuhin co., Ltd.) | 7 | 2 | 1 |

As seen from Table 16, the number of evaluators who detected a bitter taste in the peptide products treated with phospholipase D was significantly smaller than that in the peptide products without such treatment, and, thus, it was seen that the phospholipase D is excellent in the masking effect of suppressing the bitterness of peptide products.

### (Test Example 6: Bitterness Masking Effect on Modified Egg Yolk)

Egg yolk liquids R2 to R5 were prepared by mixing 5 g of sugar with 50 g of modified egg yolk treated in advance with the phospholipase A2, where phospholipase D powder (PLD: *Streptomyces cinnamoneum*-derived PLD, manufactured by Nagase ChemteX Corporation) in an amount (15000 U, 5000 U or 1500 U) shown in Table 17 was added to the egg yolk liquids R3 to R5, and no phospholipase D was added to R2. Furthermore, an egg yolk liquid R1 as a control was prepared by merely adding 5 g of sugar to 50 g of unmodified egg yolk without such treatment. The egg yolk liquids R1 to R5 were reacted at 40°C for 5 hours.

Then, 2 g of each of the obtained egg yolk liquids R1 to R5 was weighed and dissolved in 58 mL of tap water, and the liquid was stirred using a homo mixer at 5000 rpm for 5 minutes. Next, 140 mL of salad oil was added to each of the egg yolk liquids over 1 minute, and stirred using a homo mixer at 5000 rpm for 5 minutes. The obtained samples were preserved in a refrigerator for 3 days.

Nine evaluators performed taste test on the obtained samples, and the number of evaluators who detected a bitter taste and that not detected a bitter taste were counted. Table 17 shows the counting results, where the case in which a majority of evaluators detected a bitter taste is judged as "x" and the case in which a majority of evaluators did not detect a bitter taste is judged as "O".

**Table 17**

| Sample No. | Egg Yolk Liquid | Egg yolk | Sugar | PLD | Judgment of Bitter Taste |
|---|---|---|---|---|---|
| R1 | Only unmodified egg yolk (without modifying and treating of PLD) | 50g | 5g | - | ○ |
| R2 | Only modified egg yolk | 50g | 5g | - | × |
| R3 | Modified-egg yolk treated with PLD | 50g | 5g | 15,000U | ○ |
| R4 | Modified-egg yolk treated with PLD | 50g | 5g | 5,000U | ○ |
| R5 | Modified-egg yolk treated with PLD | 50g | 5g | 1,500U | ○ |

| | | | | | |
|---|---|---|---|---|---|
| -: not added | | | | | |

As seen from Table 17, contrary to the modified egg yolk liquid (sample number R2) not treated with the phospholipase D, a majority of evaluators did not detect a bitter taste in the egg yolk liquids (sample numbers R3 to R5) treated with the phospholipase D, which was a similar result to that of the unmodified egg yolk liquid (sample number R1) and, thus, it was seen that the phospholipase D is excellent in the masking effect of suppressing the bitterness of modified egg yolk. Furthermore, it was seen that a large amount of phospholipase D as in the case of the sample number R3 is not necessarily required, and, with use of a smaller amount of phospholipase D, such a masking effect is sufficiently achieved, and masking of the bitterness can be efficiently achieved.

### Industrial Applicability

The present invention can provide an enzyme preparation for suppressing bitterness of food. Food treated with the enzyme preparation for suppressing bitterness of food in accordance with the present invention tastes less bitter even when cooked after long-term preservation. The invention can further provide an enzyme preparation for suppressing bitterness of food and for, if necessary, tenderizing food.

## Claims

1. An enzyme preparation for suppressing bitterness of food, comprising phospholipase.

2. The enzyme preparation of claim 1, further comprising protease.

3. The enzyme preparation of claim 1 or 2, wherein the food is edible meat.

4. The enzyme preparation of any one of claims 1 to 3, wherein the bitterness is derived from peptide generated due to an action of protease on a material of the food.

5. The enzyme preparation of any one of claims 1 to 4, wherein the phospholipase is phospholipase D.

6. The enzyme preparation of any one of claims 1 to 5, wherein the phospholipase is contained in a proportion of 60 to 1500 units (U) with respect to 100 units (U) of the protease.

7. A method for suppressing bitterness of food, which comprises treating a food material with a food bitterness suppressor containing the enzyme preparation of any one of claims 1 to 6.

8. A method for producing a processed food product, which comprises treating a food material with a food bitterness suppressor containing the enzyme preparation of any one of claims 1 to 6.

9. A processed food product, comprising a food material treated with a food bitterness suppressor containing the enzyme preparation of any one of claims 1 to 6.
